# FASCICULE DE BREVET EUROPEEN

(11) **EP 0 934 295 B1**
(45) Date de publication et mention de la délivrance du brevet: **07.03.2001**
(21) Numéro de dépôt: 97911303.2
(22) Date de dépôt: 20.10.1997
(51) Int. Cl.: C07D 307/93, C07D 409/06, C07D 405/06, A61K 31/34, A61K 7/48, A61K 7/06

(54) **NOUVEAUX DERIVES BI-AROMATIQUES DU DIBENZOFURANNE ET LEUR UTILISATION EN MEDECINE HUMAINE OU VETERINAIRE AINSI QU'EN COSMETIQUE**
BIOAROMATISCHE DIBENZOFURANDERIVATE UND VERWENDUNG IN DER HUMAN- ODER TIERMEDIZIN UND IN DER KOSMETIK
NOVEL BI-AROMATIC DIBENZOFURAN DERIVATIVES AND THEIR USE IN HUMAN AND VETERINARY MEDICINE AND IN COSMETICS

(30) Priorité: 23.10.1996 FR 9612914
(43) Date de publication de la demande: 11.08.1999
(73) Titulaire: CENTRE INTERNATIONAL DE RECHERCHES DERMATOLOGIQUES GALDERMA - CIRD GALDERMA, F-06565 Valbonne (FR)
(72) Inventeur: CHARPENTIER, Bruno, F-06410 Biot (FR)
(74) Mandataire: Tezier Herman, Béatrice
(86) Numéro de dépôt international: FR9701878
(87) Numéro de publication internationale: WO9817659

(56) Documents cités:
- EP-A- 0 708 100
- EP-A- 0 709 382

## Description

La présente invention concerne, à titre de produits industriels nouveaux et utiles, des composés bi-aromatiques présentant à la fois un motif lipophile dérivé du 1,2,3,4,4a,9b-hexahydro-1,4a,9b-triméthyl-1,4-méthanodibenzofuranne connecté à un second noyau aromatique par l'intermédiaire d'un linker à 3 liaisons. Les composés obtenus présentent des réponses pharmacologiques de type agoniste rétinoïde, caractérisée par une action marquée dans les domaines de la différenciation et de la prolifération cellulaire.

La présente invention concerne également l'utilisation de ces nouveaux composés dans des compositions pharmaceutiques destinées à un usage en médecine humaine ou vétérinaire, ou bien encore dans des compositions cosmétiques.

Les composés selon l'invention présentant une activité dans la prolifération et la différentiation cellulaire, ils peuvent être, en conséquence utilisés dans le traitement topique et systémique des affections dermatologiques liées à un désordre de la kératinisation, des affections dermatologiques (ou autres) à composante inflammatoire, virale et/ou immunoallergique et des proliférations dermiques ou épidermiques, qu'elles soient bénignes ou malignes. Ces composés peuvent être utilisés en outre, dans le traitement des maladies de dégénérescence du tissu conjonctif, pour lutter contre le vieillissement de la peau, qu'il soit photoinduit ou chronologique et traiter les troubles de la cicatrisation. Ils trouvent enfin une application dans le domaine ophtalmologique, notamment dans le traitement des cornéopathies. Les composés selon l'invention peuvent par ailleurs trouver une application dans le domaine de l'ostéoporose. D'une manière générale, ils peuvent enfin trouver une application dans le traitement de toute maladie qui est associée à une modification de l'expression des récepteurs de la super famille des récepteurs nucléaires des hormones thyroïdiennes et stéroïdiennes.

On peut également les utiliser dans les compositions cosmétiques pour l'hygiène corporelle ou capillaire.

On connait des antagonistes de l'acide rétinoïque comprenant une partie lipophile dérivée du 1,2,3,4,4a,9b-hexahydro-1,4a,9b-trimethyl-1,4-methanodibenzofuranne et un linker comportant 4 liaisons libres ou incluses dans un cycle (EP 708 100 et EP 709 382 ).

Il est également connu jusqu'à présent, que le passage d'une structure rétinoïde agoniste à rétinoïde antagoniste, nécéssite d'introduire des modifications bien définies sur la partie lipophile ( A. Nadzan, Annual report in médicinal chemistry, 1995, vol30, 119; M. Teng, T et al. J. Medicinal Chemistry, 1996, vol39, 3035; C. Apfel et al; Proceedings of National Academy of Sciences, USA, 1992, vol 89, 7129).

Or, la Demanderesse vient maintenant de trouver de manière surprenante et contrairement à ce qui a été établi jusqu'à présent, que la seule modification du linker (motif chimique comportant un enchaînement à trois liaisons dans ce cas) permet de passer d'une activité antagoniste à agoniste, sans autres modifications par ailleurs.

Les composés selon l'invention sont représentés par la formule générale (I) suivante : 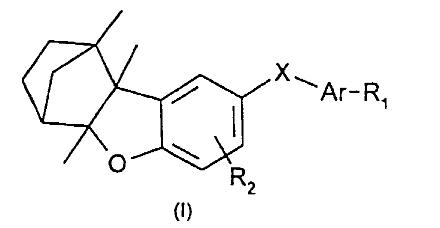 dans laquelle,
Ar représente l'un des radicaux suivants: 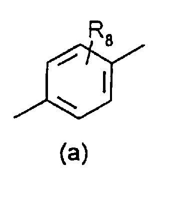 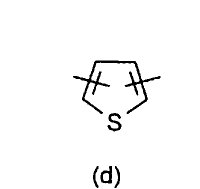 R₈ étant défini ci-dessous,
X représente les liaisons de formule (a)-(g) suivantes pouvant être lues dans les deux sens: 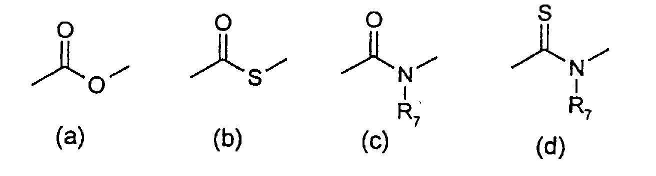 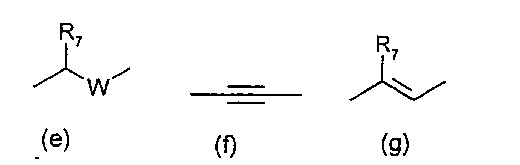 W et R₇ étant défini ci-dessous,
R₁ représente
(i) un atome d'hydrogène,
(ii) le radical -CH₃,
(iii) le radical -O-R_{3,}
(iv) le radical -CH₂-O-R_{3,}
(v) le radical -O(CH₂)ₘ-(CO)ₙ-R₄,
(vi) le radical -CO-R₅
(vii) le radical -CO-O-R₆
R₂ représente un atome d'hydrogène ou le radical -(CH₂)ₙ-O-R₃,
m et n ainsi que R₃ à R₆ étant définis ci-après,
- W représente l'atome d'oxygène ou le radical N-R₇, R₇ étant défini ci-dessous,
- R₃ représente un atome d'hydrogène, un radical alkyle ayant de 1 à 12 atomes de carbone ou un radical
   -CO-R₉, R₉ étant défini ci-dessous,
- R₄ représente un radical alkyle ayant de 1 à 12 atomes de carbone ou un hétérocycle,
- R₅ représente un atome d'hydrogène, un radical alkyle ayant de 1 à 12 atomes de carbone, ou un radical
   -N (r',r"),
   dans lequel, r' et r" identiques ou différents représentent un atome
   d'hydrogène, un radical alkyle ayant de 1 à 12 atomes de carbone, un radical mono ou polyhydroxyalkyle, un radical phényle, éventuellement substitué par un ou plusieurs atomes d'halogène, une fonction hydroxyle ou une fonction nitro, ou un groupe méthoxy ou un reste d'amino acide, de peptide ou de sucre ou encore r' et r" pris ensemble forment un hétérocycle,
- R₆ représente un atome d'hydrogène, un radical alkyle linéaire ou ramifié ayant de 1 à 20 atomes de carbone, un radical alkényle, un radical mono ou polyhydroxyalkyle, un radical aryle ou aralkyle, un reste de sucre ou d'aminoacide ou de peptide,
- R₇ représente un atome d'hydrogène, un radical alkyle ayant de 1 à 12 atomes de carbone,
- R₈ représente le radical -(CH₂)ₙ-O-R₃, R₃ étant défini ci-dessus,
- R₉ représente un atome d'hydrogène, un radical alkyle ayant de 1 à 12 atomes de carbone, un radical aryle,
- m est un nombre entier égal à 1, 2 ou 3,
- n est un nombre entier égal à 0 ou 1,
et les isomères optiques et géométriques desdits composés purs, ou leurs mélanges en toutes proportions ainsi que leurs sels pharmaceutiquement acceptables.

Les sels pharmaceutiquement acceptables sont en particulier les sels d'addition d'un acide ou d'une base usuels. Lorsque les composés selon l'invention se présentent sous forme de sels par addition d'une base il s'agit de sels d'un métal alcalin ou alcalino-terreux ou encore de zinc ou d'une amine organique. Lorsque les composés se présentent sous forme de sels, par addition un acide, il s'agit de sels pharmaceutiquement ou cosmétiquement acceptables obtenus par addition d'un acide minéral ou organique, en particulier l'acide chlorhydrique, sulfurique, acétique, citrique, fumarique, hémisuccinique, maléique et mandélique.

Par radical alkyle inférieur, on entend un radical ayant de 1 à 12 atomes de carbone, pouvant être linéaire ou ramifié et de préférence, les radicaux méthyle, éthyle, isopropyle, butyle et tertiobutyle,hexyle, nonyle et dodecyle.

Par radical alkyle, on entend un radical linéaire ou ramifié ayant de 1 à 20 atomes de carbone, notamment les radicaux méthyle, éthyle, isopropyle, butyle et tertiobutyle,hexyle, nonyle et dodecyle, hexadecyle et octadecyle.

Par radical alcenyle, on entend un radical ayant de 1 à 20 atomes de carbone, linéaire ou ramifié présentant une double liaison.

Par radical monohydroxyalkyle, on doit entendre un radical ayant de 1 à 6 atomes de carbone, notamment un radical 2-hydroxyéthyle, 2-hydroxypropyle ou 3-hydroxypropyle.

Par radical polyhydroxyalkyle, on doit entendre un radical contenant de 2 à 6 atomes de carbone et de 2 à 5 groupes hydroxyles tels que les radicaux 2,3-dihydroxypropyle, 2,3,4-trihydroxybutyle, 2,3,4,5-tétrahydroxypentyle ou le reste du pentaérythritol.

Par radical aryle éventuellement substitué, on doit entendre un radical phényle, éventuellement substitué par un ou plusieurs atomes d'halogène, une fonction hydroxyle ou fonction nitro, ou un groupe méthoxy.

Par reste d'aminoacide, on doit entendre un reste dérivant par exemple de l'un des 20 aminoacides de configuration L ou D constitutifs de protéines de mammifères. Par reste de peptide, on doit entendre un peptide linéaire de 2 à 10 acides aminés.

Par reste de sucre on doit entendre un reste dérivant par exemple du glucose, du galactose, du mannose ou de l'acide glucuronique.

Par hétérocycle, on entend de préférence un radical pipéridino, morpholino, pyrrolidino ou pipérazino, éventuellement substitué en position 4 par un radical alkyle en C₁-C₆ ou mono ou polyhydroxyalkyle tels que définis ci-dessus.

Parmi les composés de formule (I) ci-dessus, rentrant dans le cadre de la présente invention, on peut notamment citer les suivants:
2-[(E)-2-(1,2,3,4,4a, 9b-Hexahydro-1,4a,9b-triméthyl-1,4-méthanodibenzofuranne-8-yl)-1-propenyl] -4-thiophène carboxylate d'éthyle
Acide 2-[(E)-2-(1,2,3,4,4a,9b-Hexahydro-1,4a,9b-triméthyl-1,4-méthanodibenzofuranne -8-yl)-1-propenyl] 4-thiophène carboxylique
Acide 2-[(E)-2-(1,2,3,4,4a,9b-Hexahydro-1,4a,9b-triméthyl-1,4-méthanodibenzofuranne -8-yl)-1-propenyl] -5-thiophène carboxylique
4-[3-(1,2,3,4,4a,9b-Hexahydro-1,4a,9b-triméthyl-1,4-méthanodibenzofuranne -8-yl) éthynyl] benzoate de méthyle
Acide 4-[3-(1,2,3,4,4a,9b-Hexahydro-1,4a,9b-triméthyl-1,4-méthanodibenzofuranne -8-yl) éthynyl] benzoïque
Acide 4-(-2-(1,2,3,4,4a,9b-Hexahydro-1,4a,9b-triméthyl-1,4-méthanodibenzofuranne -8-yl) éthynyl] -2-thiophéne carboxylique.
4-[3-(1,2,3,4,4a,9b-Hexahydro-1,4a,9b-triméthyl-1,4-méthanodibenzofuranne-8-yl) carbonyloxy] benzoate de méthyle
Acide 4-[3-(1,2,3,4,4a,9b-Hexahydro-1,4a,9b-triméthyl-1,4-méthanodibenzofuranne -8-yl) carbonyloxy] benzoïque
4-[(1,2,3,4,4a,9b-Hexahydro-1,4a,9b-triméthyl-1,4-méthanodibenzofuranne -8-yl) carboxamido] benzoate de benzyle
Acide 4-[(1,2,3,4,4a,9b-Hexahydro-1,4a,9b-triméthyl-1,4-méthanodibenzofuranne-8-yl) carboxamido] benzoïque
4 -[(1,2,3,4,4a,9b-Hexahydro-1,4a,9b-triméthyl-1,4-méthanodibenzofuranne-8-yl) carboxamido] -2-hydroxybenzoate de méthyle
Acide 4-[(1,2,3,4,4a,9b-Hexahydro-1,4a,9b-triméthyl-1,4-méthanodibenzofuranne-8-yl) carboxamido] -2-hydroxy benzoïque
Acide 4-[(1,2,3,4,4a,9b-Hexahydro-1,4a,9b-triméthyl-1,4-méthanodibenzofuranne-8-yl) thiocarboxamido] benzoate de méthyle
Acide 4-[(1,2,3,4,4a,9b-Hexahydro-1,4a,9b-triméthyl-1,4-méthanodibenzofuranne-8-yl) thiocarboxamido] benzoïque

Parmi les composés de formule (I) ci-dessus, on préfère fes composés répondant à la formule générale (la) suivante 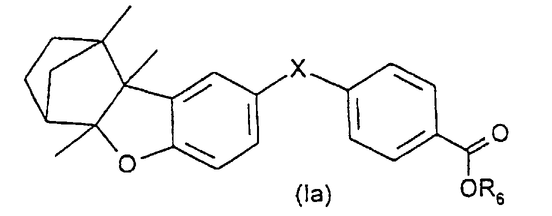 dans laquelle,
X a la signification (c), (d), (f) ou (g),
(c), (d), (f), (g), et R₆ étant définis précédemment.

Dans la suite, nous illustrons les méthodes de préparation des composés de formule générale (I).

Les composés de formule Ib sont préparés à partir des dérivés aldéhydiques ou cétoniques de formule II selon une réaction de Wittig ou de Horner-Emmons comme indique dans le schéma ci-dessous:

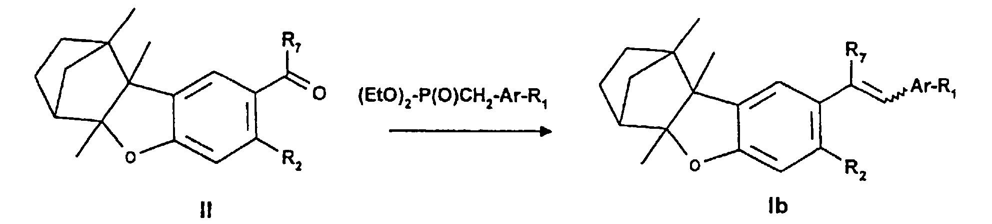

Les dérivés acétyléniques lc peuvent être préparés selon trois méthodes différentes, comme indiqué sur la figure 1 ci-dessous:
- soit par transformation du dérivé bromé III en composé aldéhydique IV, puis conversion de l'aldéhyde en dérivé dibromostyrène V avec du Tétrabromure de carbone et de la triphénylphosphine .Ce composé est alors converti en triple liaison par action d'une base telle que le n-butyl lithium dans un solvant aprotique tel que le tétrahydrofuranne;
- soit par transformation du dérivé bromé III en dérivé triméthylsilylacétylénique VI, puis désilylation en présence soit de carbonate de potassium, soit de fluorure de tétrabutylamonium.
- soit à partir du dérivé méthyl cetone X qui est alors traitée par du diisopropylamidure de lithium (LDA), puis un chlorure de dialkylphosphate et de nouveau avec de la LDA:

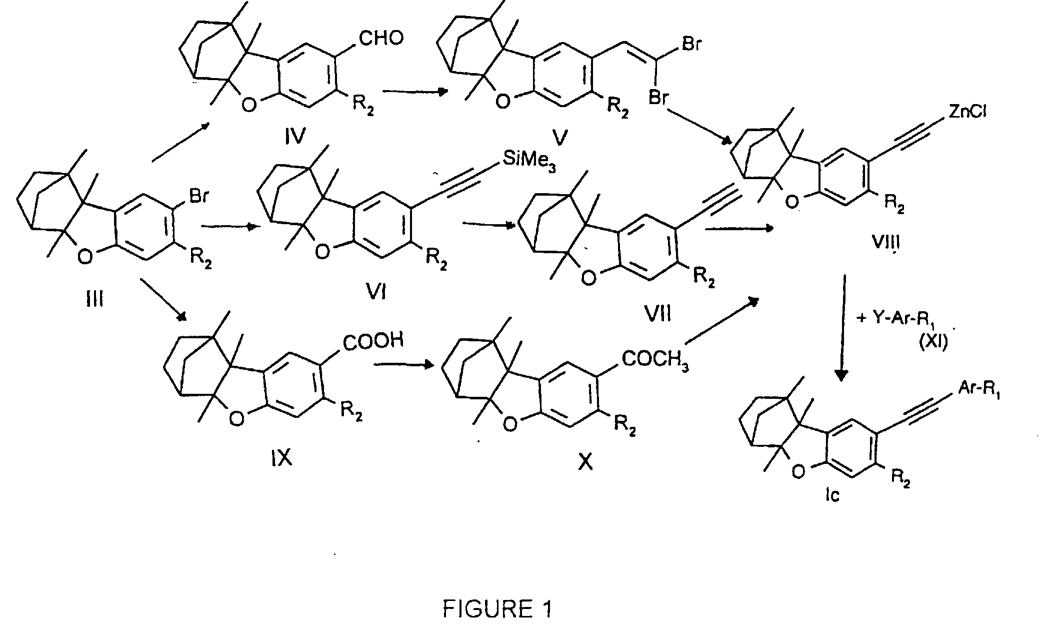

Le dérivé acetylénique VII est alors converti en organolithien puis en organozincique VIII et condensé sur un dérivé halogéné (iodé ou bromé) IX en présence d'un catalyseur au palladium tel que le Tétrakis(triphenylphosphine)palladium (0), dans un solvant tel que le THF, selon les conditions décrites par Negishi et al. (J.Org.Chem.1977, vol.42,1821-1823).

Les composés de formule Id, le, If et Ig sont obtenus selon les voies de synthèse décrites sur la figure 2 ci-dessous.

Les composés de formule Id peuvent être obtenus en faisant réagir en milieu anhydre , dans un solvant organique tel que le THF, en présence d'une amine tertiaire, une forme activée de l'acide benzoïque telle qu'un chlorure d'acide XII, par exemple sur un composé phénolique XIII .

Les composés de formule le peuvent être obtenus dans les mêmes conditions que pour la préparation des composés Id en remplaçant le dérivé phénolique par un dérivé thiophénolique XIV.

Les composés de structure If sont obtenus dans les mêmes conditions que pour la préparation des dérivés Id en remplaçant la dérivé phénolique XIII par un dérivé arylaminé XV.

Les composés de formule Ig peuvent être obtenus à partir des composés de formule If par réaction avec le réactif de Lawesson.

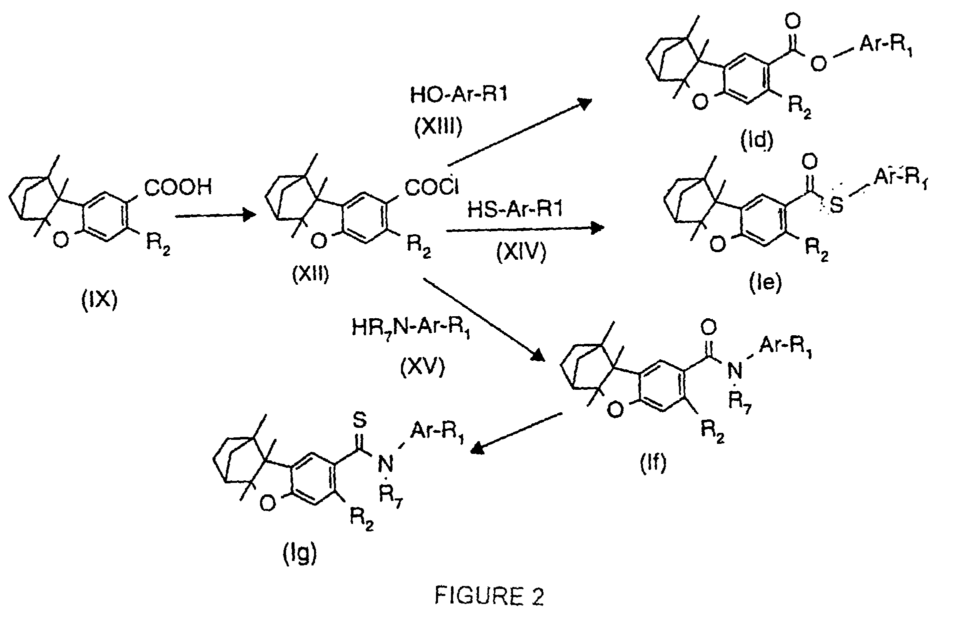

Les composés de formule Ih peuvent être obtenus à partir des dérivés bromés de formule XVI avec un composé phénol, thiophénol ou aminé de formule XVII en présence de carbonate de potassium ou d'un hydrure alcalin tel que l'hydrure de sodium ou par transfert de phase.

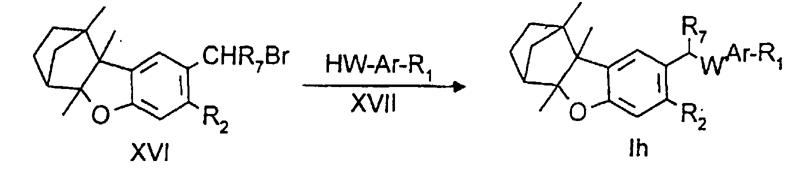

Au cours de toutes ces réactions ci-avant mentionnées, R₁, R₂, R₇ et R₉ ont les significations décrites dans la formule I ou sont des groupements protecteurs des groupes fonctionnels, compatibles avec les conditions réactionnelles. Les groupements protecteurs employés sont ceux décrits dans le livre "Protecting groups in organic synthesis" by T.W. Greene, Ed. by John Wiley and Sons (1981).

Pour la préparation des composés de formule générale I, on pourra être amenés à procéder à des réactions classiques de la chimie organique telles que, par exemple,celles décrites ci-dessous en faisant appel aux méthodes décrites dans le "Advanced Organic Chemistry" de J. March; John Wiley and Sons, 1985. Certaines transformations classiques de transformation de fonctions sont présentées ci-dessous:
acide carboxylique → ester
ester → acide carboxylique
acide → chlorure d'acide
chlorure d'acide → amide
acide → amide
acide → alcool
alcool → aldéhyde
amide → amine
thiol → thioéther
thioéther → sulfoxyde
thioéther → sulfone

Ces composés présentent une activité agoniste vis à vis de l'expression d'un ou plusieurs marqueurs biologiques dans le test de différenciation des cellules (F9) de tératocarcinome embryonnaire de souris (Skin Pharmacol. 3, p.256-267, 1990) et/ou sur la différenciation des kératinocytes humains in vitro (Skin Pharmacol. 3 p.70-85, 1990) . Leurs activités sur ces récepteurs peuvent aussi être mesurées dans des tests de transactivation cellulaire à l'aide de récepteurs recombinants RARs ou RXRs préalablement transfectés. ( Biochemical and Biophysical Research Communication 1992, vol. 186, 977-983; Journal of Medicinal Chemistry, 1994, 37, 408-414). Les composés de cette série peuvent présenter suivant la nature du linker et du noyau aromatique, une sélectivité pour le sous-type de récepteurs RAR alpha. Leurs affinités pour les récepteurs nucléaires de l'acide rétinoïque (RARs) sont mesurées selon la méthode précédemment décrite ( Journal of Medicinal Chemistry, 1995, vol.38, 4993-5006).

Ces composés sont aussi caractérisés par des activités pharmacologiques dans le test d'inhibition de l'ornithine decarboxylase Aprèsinduction par le TPA chez la souris (Cancer Research, 1978, 38, 793-801).

Les composés selon l'invention conviennent particulièrement bien dans les domaines de traitement suivants :
1) Pour traiter les affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle.
2) Pour traiter d'autres types de troubles de la kératinisation, notamment les ichtyoses, les états ichtyosiformes, la maladie de Darier, les kératodermies palmoplantaires, les leucoplasies et les états leucoplasiformes, le lichen cutané ou muqueux (buccal).
3) Pour traiter d'autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale; les composés peuvent également être utilisés dans certaines affections inflammatoires ne présentant pas de trouble de la kératinisation.
4) Pour traiter toutes les proliférations dermiques ou épidermiques qu'elles soient bénignes ou malignes, qu'elles soient ou non d'origine virale telles que les verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires.
5) Pour traiter d'autres désordres dermatologiques tels que les dermatoses bulleuses et les maladies du collagène.
6) Pour traiter certains troubles ophtalmologiques, notamment les cornéopathies.
7) Pour réparer ou lutter contre le vieillissement de la peau, qu'il soit photoinduit ou chronologique, ou pour réduire les pigmentations et les kératoses actiniques, ou toutes pathologies associées au vieillissement chronologique ou actinique.
8) Pour prévenir ou guérir les stigmates de l'atrophie épidermique et/ou dermique induite par les corticostéroïdes locaux ou systémiques, ou tout autre forme d'atrophie cutanée.
9) Pour prévenir ou traiter les troubles de la cicatrisation ou pour prévenir ou réparer les vergetures.
10) Pour lutter contre les troubles de la fonction sébacée tels que l'hyperséborrhée de l'acné ou la séborrhée simple.
11) Dans le traitement ou la prévention des états cancéreux ou précancéreux, plus particulièrement les leucémies promyélocytaires.
12) Dans le traitement d'affections inflammatoires telles que l'arthrite.
13) Dans le traitement de toute affection d'origine virale au niveau cutané, telle que le syndrome de Kaposis, ou général.
14) Dans la prévention ou le traitement de l'alopécie.
15) Dans le traitement d'affections dermatologiques à composante immunitaire.
16) Dans le traitement d'affections du système cardiovasculaire telles que l'artériosclérose ou l'hypertension ainsi que le diabète non-insulino dépendant.
17) dans le traitement de désordres cutanés dus à une exposition aux rayonnements U.V..

Dans les domaines thérapeutiques mentionnés ci-dessus, les composés selon l'invention peuvent être avantageusement employés en combinaison avec d'autres rétinoïdes, avec les vitamines D ou leurs dérivés, avec des corticostéroïdes, en association avec des antioxydants, avec des a-hydroxy ou a-céto acides ou leurs dérivés, ou encore avec des bloqueurs de canaux ioniques.

Par vitamines D ou leurs dérivés, on entend par exemple les dérivés de la vitamine D₂ ou D₃ et en particulier la 1,25-dihydroxyvitamine D₃.

Par antioxydants, on entend par exemple l'a-tocophérol ou ses dérivés, les flavonoïdes, les antioxydants du type BHT, BHA ou leurs dérivés, l'acide ascorbique ou encore certains chélatants de métaux.

Par α-hydroxy ou α-céto acides ou leurs dérivés, on entend par exemple les acides lactique, malique, citrique, glycolique, mandélique, tartrique, glycérique ou ascorbique ou dérivés de l'acide salicylique ou leurs sels, amides ou esters.

Par bloqueurs de canaux ioniques, on entend par exemple le Minoxidil (2,4-diamino-6-pipéridino-pyrimidine-3-oxyde) et ses dérivés.

La présente invention a également pour objet des compositions médicamenteuses contenant au moins un composé de formule (I) telle que définie ci-dessus, l'un de ses isomères optiques ou géométriques ou un de ses sels.

La présente invention a donc aussi pour objet une nouvelle composition médicamenteuse destinée notamment au traitement des affections susmentionées, caractérisée par le fait qu'elle comporte, dans un support pharmaceutiquement acceptable au moins un composé de formule (I), telle que définie ci-dessus.

L'administration des composés selon l'invention peut être effectuée par voie entérale, parentérale, topique ou oculaire.

Par voie entérale, les médicaments peuvent se présenter sous formes de comprimés, de gélules, de dragées, de sirops, de suspensions, de solutions, de poudres, de granulés, d'émulsions, de microsphères ou nanosphères ou vésicules lipidiques ou polymériques permettant une libération contrôlée. Par voie parentérale, les compositions peuvent se présenter sous forme de solutions ou suspensions pour perfusion ou pour injection.

Les composés selon l'invention sont généralement administrés à une dose journalière d'environ 0,01 mg/kg à 100 mg/kg en poids corporel en 1 à 3 prises.

Par voie topique, les compositions pharmaceutiques à base de composés selon l'invention sont destinées au traitement de la peau et des muqueuses et se présentent sous forme d'onguents, de crèmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent se présenter soit sous forme anhydre, soit sous forme aqueuse selon l'indication clinique.

Par voie oculaire, ce sont principalement des collyres.

Ces compositions pharmaceutiques, en particulier pour la voie topique ou oculaire contiennent au moins un composé de formule (I) telle que définie ci-dessus, l'un de ses isomères optiques ou géométriques ou un de ses sels, à une concentration de préférence comprise entre 0,001 et 5 % en poids par rapport au poids total de la composition.

Les composés de formule (I), selon l'invention, trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les effets néfastes du soleil ou dans le traitement des peaux physiologiquement sèches, pour prévenir et/ou lutter contre le vieillissement photo-induit ou chronologique.

Dans le domaine cosmétique, les composés selon l'invention peuvent être avantageusement employés en combinaison avec d'autres rétinoïdes, avec les vitamines D ou leurs dérivés, avec des corticostéroïdes, en association avec des anti-radicaux libres, avec des α-hydroxy ou α-céto acides ou leurs dérivés, ou encore avec des bloqueurs de canaux ioniques.

Les différents produits pris en association avec les composés de la présente invention étant tels que définis ci-dessus.

La présente invention vise donc également une composition cosmétique contenant, dans un support cosmétiquement acceptable, au moins un composé de formule (I) telle que définie ci-dessus, cette composition se présentant notamment sous forme d'une crème, d'un lait, d'une lotion, d'un gel, de microsphères ou nanosphères ou vésicules lipidiques ou polymériques, d'un savon ou d'un shampooing.

La concentration en composé de formule (I), dans les compositions cosmétiques est comprise entre 0,001 et 3 % en poids par rapport au poids total de la composition.

Les compositions médicamenteuses et cosmétiques selon l'invention peuvent, en outre, contenir des additifs inertes ou même pharmacodynamiquement ou cosmétiquement actifs ou des combinaisons de ces additifs et notamment : des agents mouillants; des agents dépigmentants tels que l'hydroquinone, l'acide azelaïque, l'acide caféïque ou l'acide kojique; des émollients; des agents hydratants comme le glycérol, le PEG 400, la thiamorpholinone et ses dérivés ou l'urée; des agents antiséborrhéiques ou antiacnéiques, tels que la S-carboxyméthylcystéine, la S-benzyl-cystéamine, leurs sels et leurs dérivés, ou le péroxyde de benzoyle; des antibiotiques comme l'érythromycine et ses esters, la néomycine, la clindamycine et ses esters, les tétracyclines; des agents antifongiques tels que le kétoconazole ou les polyméthylène-4,5 isothiazolinones-3 ; des agents favorisant la repousse des cheveux, comme le Minoxidil (2,4-diamino-6-pipérjdino-pyrimidine-3-oxyde) et ses dérivés, le Diazoxide (7-chloro 3-méthyl 1,2,4- benzothiadiazine 1,1-dioxyde) et le Phénytoïn (5,4-diphényl-imidazolidine 2,4-dione) ; des agents anti-inflammatoires non stéroïdiens; des caroténoïdes et, notamment le b-carotène; des agents anti-psoriatiques tels que l'anthraline et ses dérivés et enfin, les acides eicosa-5,8,11,14-tétraynoïque et eicosa-5,8,11-trynoïque, leurs esters et les amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des anti-oxydants, tels que l'a-tocophérol, le butylhydroxyanisole ou le butylhydroxytoluène.

On va maintenant donner, à titre d'illustration et sans aucun caractère limitatif, plusieurs exemples de préparation des composés actifs de formule (I) selon l'invention ainsi que des exemples de compositions les contenant.

### A. EXEMPLES DE COMPOSES

Tous les produits dont les synthèses sont décrites ci-après ont été caractérisés par RMN du proton (250 MHz), spectrométrie masse et analyse élémentaire.

### Exemple 1a: Acide 2-Carboxaldehyde-4-thiophènecarboxylique

A une solution de 2-carboxaldehyde-4-bromothiophène (53g, 277mmol) dans 70 ml de benzène, on ajoute 19.4 ml d'éthylène glycol et 20mg d'acide paratoluene sulfonique.Le mélange réactionnel est porté à reflux pendant 10 heures et l'eau formée est éliminée à l'aide d'un dean stark. Après refroidissement, la phase organique est neutralisée par une solution saturée de bicarbonate .Après traitement, on isole 64.3g (99%) de 2-carboxaldehyde-4-thiophène éthylene ketal sous forme d'une huile orangée qui est alors placée en solution dans 600ml d'éther éthylique .On ajoute à cette solution à -70° , 131ml de butyl lithium(2,5M dans l'hexane) puis fait barboter du gaz carbonique dans le milieu réactionnel pendant 1h30mn et laisse revenir à température ambiante sous agitation pendant la nuit. Le milieu réactionnel est versé dans l'eau et acidifié à pH 1 par HCI 6N. Cette phase aqueuse acide est laissée sous agitation à température ambiante durant la nuit.Après extraction par de l'acétate d'éthyle, suivie par un traitement habituel et une recristallisation dans un mélange hexane/Et2O, on isole 33g(77%) du dérivé attendu sous forme de poudre blanche fondant à 163-165°C.

### Exemple 1b: 4-Ethoxycarbonyl-2-thiophènecarboxaldehyde

33g(211mmoles) de l'acide obtenu en **1a**,en solution dans 1l de DMF sont traités à température ambiante par 7g d'hydrure de sodium (à 80% dans l'huile) sous agitation durant 30 mn. On ajoute alors goutte à goutte 19ml (23,2 mmol) d'iodure de méthyle et laisse sous agitation à température ambiante pendant la nuit.Le milieu réactionnel est versé dans de l'eau glacée, puis est extrait par AcOEt. Aprèstraitement habituel suivi d'une chromatographie (hexanne/CH2CI2, 50:50),puis d'une recristallisation dans l'hexane, on isole 28,6 g (74%) du dérivé **1b** sous forme de poudre blanche fondant à 47°C.

### Exemple 1c: Ethyle 4-hydroxyméthyle-2-thiophènecarboxylate

Le composé **1b** (155mmoles) en solution dans 300ml d'un mélange éthanol-THF (1:1) est traité à température ambiante par 2.93g de borohydrure de sodium. Le milieu réactionnel est laissé sous agitation à température ambiante pendanr 2 heures.Après évaporation des solvants, addition d'eau et extraction par de l'éther, on rince la phase organique par de l'eau acidulée puis effectue le traitement habituel. On isole 27.7g du dérivé **1c** sous forme d'une huile jaune.

### Exemple 1d: Ethyle 4-bromométhyle-2-thiophènecarboxylate

Le composé **1c** (27.5g, 147mmoles) en solution dans du toluène (280ml) et de la pyridine (12ml) est traité à 0°C par addition goutte à goutte de tribromure de phosphore (16,7ml, 177mmoles) dans 30ml de toluène. Le milieu réactionnel est laissé sous agitation à température ambiante pendant la nuit. Après évaporation du toluène et addition d'eau ,on extrait la phase aqueuse par de l'acétate d'éthyle, la lave avec une solution saturée de bicarbonate, la rince,la sèche et l'évapore. Après chromatographie (hexane/CH2Cl2, 50:50),on isole 32,1g(88%) du dérivé **1d** sous forme d'huile jaunâtre.

### Exemple 1e: Diéthyle 2-ethoxycarbonyl-4-thiophèneméthylphosphonate

Le dérivé **1d** est traité par 25ml de triéthylphosphite et est chauffé à 110°C pendant 5heures. Après chromatographie (CH2Cl2/Et2O, 90:10), on isole 38.7g(98%) du dérivé **1e** sous forme d'une huile jaune.

### Exemple 2: Isomères géométriques Z et E du [2-(1,2,3,4,4a,9b-Hexahydro-1,4a,9b-triméthyl-1,4-méthanodibenzofuranne -8-yl)-1-propenyl ]-4-thiophène carboxylate d'éthyle

Dans un ballon contenant une suspension de 233mg de NaH(80% dans l'huile), dans 2ml de THF, on ajoute goutte à goutte le mélange constitué par (1,74g, 6,43 mmoles) de (-) 1,2,3,4,4a,9b-hexahydro-1,4a,9b-triméthyl-1,4-méthano dibenzofuranne-8-yl, méthylcétone décrite dans EP 709 382, le phosphonate obtenu à l'exemple **1e** et 280mg d'éther couronne (15crown5) dans 40ml de THF. La réaction est laissée sous agitation à température ambiante pendant la nuit. Le milieu réactionnel est versé dans de l'eau glacée,est acidifié par de l'acide chlorydrique 6N et est extrait par de l'éther éthylique. Après un traitement habituel, suivi par une chromatographie (hexane/CH2Cl2, 70:30), on isole 1.67g du mélange des isomères géométriques Z et E de l'éthyl [2-(1,2,3,4,4a,9b-hexahydro-1,4a,9b-triméthyl-1,4-méthano dibenzofuranne-8-yl)-1-propenyl] 4-thiophène carboxylate sous forme d'une huile jaunâtre.

### Exemple3: (-) Acide 2-[(Z)-2-(1,2,3,4,4a,9b-Hexahydro-1,4a,9b-triméthyl-1,4-méthano dibenzofuranne-8-yl)-1-propenyl ] -4-thiophène carboxylique

1,61g (3,8 mmoles du mélange d'esters obtenus à l'exemple **2** en solution dans 30ml d'EtOH sont traités par 1,68g d'hydroxyde de sodium et chauffés 1heure à reflux . Aprèsévaporation de l'éthanol et addition d'eau, on acidifie le milieu par de l'HCl 2N et extrait par de l'éther éthylique.Le traitement habituel suivi d'une chromatographie HPLC préparative (colonne C18,éluent CH3CN/0,1% d'acide trifluoroacetique dans l'eau, 65:35) 0,62g (42%) du dérivé attendu **3** sous forme d'une poudre blanche fondant à 220-222°C; α_{D}= -45° (c=1,DMF).

### Exemple 4: (+)Acide 2-[(E)-2-(1,2,3,4,4a,9b-Hexahydro-1,4a,9b-triméthyl-1,4-méthano dibenzofuranne-8-yl)-1-propenyl]-4-thiophène carboxylique

La chromatographie HPLC conduite sur le mélange d'acides obtenus à l'exemple **3** permet aussi d'obtenir 0.27g (18%) de l'isomère E sous forme d'une poudre blanche fondant à 200°C; αD=+17,3° (c= 1, DMF).

### Exemple 5: (+) Acide 2-[(Z)-2-(1,2,3,4,4a,9b-Hexahydro-1,4a,9b-triméthyl-1,4-méthano dibenzofuranne -8-yl)-1-propenyl ] -4-thiophène carboxylique

2,07g (7.65mmoles) de (+) [1,2,3,4,4a,9b-hexahydro-1,4a,9b-triméthyl-1,4-méthanodibenzofuranne-8-yl, méthylcétone ] sont traités par du diéthyle 4-bromométhyle-2-thiophènecarboxylate (2,81g, 9,18mmoles) dans les conditions décrites à l'exemple **2**. On isole après chromatographie sur silice 1,41g (68%) du mélange Z/E des composés éthyl [2-(1,2,3,4,4a,9b-Hexahydro-1,4a,9b-triméthyl-1,4-méthano dibenzofuranne -8-yl)-1-propenyl]-4-thiophène carboxylate sous forme d'une poudre blanche. Ce mélange est saponifié et purifié par HPLC dans les conditions décrites à l'exemple 3. On isole alors 0,3g (14%) du composé 5 sous forme d'une poudre blanche fondant à 187-189°C; α_{D}= + 48° (c=1, DMF).

### Exemple 6: (-) Acide 2-[(E)-2-(1,2,3,4,4a,9b-Hexahydro-1,4a,9b-triméthyl-1,4-méthano dibenzofuranne -8-yl)-1-propenyl ]-4-thiophènecarboxylique

La purification HPLC sur le mélange d'acides obtenus à l'exemple 5 permet aussi d'obtenir 0,7g du dérivé **6** sous forme d'une poudre blanche fondant à 212-214°C; α_{D}= - 15° (c=1, DMF).

### Exemple 7: 4-[-2-(1,2,3,4,4a,9b-Hexahydro-1,4a,9b-triméthyl-1,4-méthano dibenzofuranne -8-yl)-éthynyl] benzoate de méthyle

A une solution de 2,8g (11mmoles) du composé (-) 8-éthynyl-(1,2,3,4,4a,9b-Hexahydro-1,4a,9b-triméthyl-1,4-méthano dibenzofuranne) décrit dans EP 709 382 dans 30ml de THF à 0°C, on ajoute, goutte à goutte 7,6ml de n-butyl lithium(1,6M) dans l'hexane et agite 30minutes à température ambiante On refroidit alors à 0°C et ajoute 1,66g (12mmoles) de ZnCI2, puis agite 1 heure à température ambiante.

Dans un tricol contenant 30ml de THF , on introduit 2,56g (2,2mmoles) de Tétrakis(triphenylphosphine)palladium(0) et 2,91g (11mmoles) de 4-iodobenzoate de méthyle. Cette solution est agitée à température ambiante durant 30minutes, puis, on lui additionne la solution d'organozincique préparée préalablement.Le mélange réactionnel est agité à température ambiante durant 4 jours.le milieu réactionnel est versé dans 200ml d'acide chlorhydrique 3N à 0°C Après extraction à l'éther de ce milieu suivie du traitement habituel et d'une chromatographie sur silice (hexane/dichlorométhane,80:20), on isole 2,34g (55%) du dérivé attendu, fondant à 155-156°C.

### Exemple 8 : (+) Acide 4-[-2-(1,2,3,4,4a,9b-Hexahydro-1,4a,9b-triméthyl-1,4-méthano dibenzofuranne-8-yl) éthynyl] benzoique

2,33g (6mmoles) de l'ester obtenu à l'exemple 7 dans 250ml de methanol contenant 2,31g de soude, sont chauffés à reflux pendant 5 heures. Après le même traitement qu'à l'exemple **3**, suivi d'une recristallisation dans le mélange acatate d'éthyle-hexanne, on isole 1,17g (68%) du dérivé **8** sous forme d'une poudre blanche fondant à 253-255°C; α_{D} = +16,3(c=1, DMF).

### Exemple 9: 4-[-2-(1,2,3,4,4a,9b-Hexahydro-1,4a,9b-triméthyl-1,4-méthano dibenzofuranne -8-yl) -éthynyl] -2-thiophène carboxylate d'éthyle.

2,1g (8,3mmoles)de l'alcyne (-) 8-éthynyl-1,2,3,4,4a,9b-Hexahydro-1,4a,9b-triméthyl-1,4-méthano dibenzofuranne décrit dans EP 709 382 sont convertis en organozincique et sont condensés avec 1,95g (8,3mmoles) de 4-bromo-2-thiophènecarboxylate d'éthyle dans les conditions décrites à l'exemple **7**. Après le même traitement que pour l'isolement du composé **7**, suivi d'une chromatographie sur silice(hexane-dichlorométhane, 70:30),on isole 0,98g(29%) du dérivé **9** sous forme d'une huile jaune.

### Exemple 10: (+) Acide 4-[-2-(1,2,3,4,4a,9b-Hexahydro-1,4a,9b-triméthyl-1,4-méthano dibenzofuranne -8-yl) éthynyl] -2-thiophène carboxylique.

Le composé obtenu à l'exemple **9** (0,96g, 2,36mmoles) est saponifié en présence de 0,95g de soude dans les conditions décrites à l'exemple **8**. On isole Aprèsle même traitement, suivi d'une recristallisation dans un mélange éther-hexane,0,70g (79%) du dérivé attendu, sous forme d'une poudre blanche, fondant à 203-205°C; α_{D} = +22,2(c=1, CHCl3).

### Exemple 11: 4-[(1,2,3,4,4a,9b-Hexahydro-1,4a,9b-triméthyl-1,4-méthano dibenzofuranne -8-yl) carbonyloxy] benzoate de benzyle

2,54g (9,3mmoles) de l'acide (-) [1,2,3,4,4a,9b-Hexahydro-1,4a,9b-triméthyl-1,4-méthano dibenzofuranne -8-yl] carboxylique décrit dans EP 709 382 dans 50ml de toluène sont traités par 1,35ml de chlorure de thionyle et sont chauffés à reflux pendant 4 heures. Après évaporation du solvant, on isole 4,6g de chlorure de l'acide (1,2,3,4,4a,9b-Hexahydro-1,4a,9b-triméthyl-1,4-méthanodibenzofuranne-8-yl) carboxylique sous forme d'une poudre beige.

2,15g (9,4mmoles) de parahydroxybenzoate de benzyle dans 20ml de THF sont traités par 0,31g d'hydrure de sodium (80% dans l'huile) .Ce mélange réactionnel est alors soumis à une addition goutte à goutte du chlorure d'acide en solution dand 30ml de THF. Après addition, on laisse sous agitation à température ambiante pendant la nuit. Le milieu réactionnel est alors versé dans l'eau glacée et extrait à l'éther. Après le traitement habituel, suivi d'une chromatographie sur silice (hexane/dichlorométhane, 40:60), on isole 4g (91%) du dérivé **11** sous forme de cristaux blancs fondant à 131-133°C.

### Exemple 12: (+) Acide 4-[(1,2,3,4,4a,9b-Hexahydro-1,4a,9b-triméthyl-1,4-méthano dibenzofuranne -8-yl) carbonyloxy] benzoïque

L'ester benzylique **11** (4g, 8,3mmoles) en solution dans 50ml de dioxanne est hydrogéné en présence de 0,8g de palladium sur charbon (10%) sous 5 bars d'hydrogène pendant 1heure 30. Le milieu réactionnel est alors filtré sur célite puis évaporé pour conduire à 3,15g (97%) du dérivé **12** sous forme de solide cristallin blanc ,fondant à 222-224°C; α_{D} = +3,7°(c=1, CHCl3).

### Exemple 13: (-) Acide 4-[(1,2,3,4,4a,9b-Hexahydro-1,4a,9b-triméthyl-1,4-méthano dibenzofuranne -8-yl) carbonyloxy] benzoïque

La même synthèse que celle effectuée dans le cas de l'exemple **11** à partir de l'acide (+) (1,2,3,4,4a,9b-Hexahydro-1,4a,9b-triméthyl-1,4-méthano dibenzofuranne -8-yl) carboxylique, suivie d'une hydrogénation dans les mêmes conditions que pour l'exemple 21 conduit à l'énantiomère lévogyre **13** présentant les mêmes caractéristiques physico-chimiques que celles de l'énantiomère **12** ;α_{D} =-3,2°(c=1, CHCl3).

### Exemple 14: 4-[(1,2,3,4,4a,9b-Hexahydro-1,4a,9b-triméthyl-1,4-méthano dibenzofuranne -8-yl) carboxamido] benzoate de benzyle

4g (14,8 mmoles) de l'acide (-) [1,2,3,4-tétrahydro-1,4a,9b-triméthyl-1,4-méthano dibenzofuranne -8-yl] carboxylique décrit dans EP 709 382 sont convertis en chlorure d'acide dans les conditions décrites à l'exemple **11**. Le chlorure d'acide résultant est placé dans 50ml de THF puis est traité par addition goutte à goutte d'une solution contenant 3,36g (14,8 mmoles) de para aminobenzoate de benzyle, 2,3ml de triéthylamine et 20mg de 4-N,Ndiméthylaminopyridine. Le milieu réactionnel est alors laissé sous agitation à température ambiante pendant la nuit. Ce milieu est alors versé dans de l'eau glacée, puis est extrait par de l'éther. Le traitement habituel, suivi d'une chromatographie sur silice (hexane/dichlorométhane, 20:80) et d'une recristallisation dans l'hexane conduit à 6,4g (90%) du dérivé **14** sous forme de cristaux blancs fondant à 74-76°C.

### Exemple 15: (+) Acide 4-[(1,2,3,4,4a,9b-Hexahydro-1,4a,9b-triméthyl-1,4-méthano dibenzofuranne -8-yl) carboxamido] benzoïque

6,4g (13mmoles) du dérivé obtenu à l'exemple **14** en solution dans 60ml de dioxanne sont hydrogénés sous une pression d'hydrogène de 5 Bars à température ambiante durant 1 heure. Après le même traitement qu'à l'exemple **12**, suivi d'une chromatographie (dichlorométhane/méthanol, 9:1), on isole 3,8g (75%) du dérivé 15 sous forme d'une poudre blanche fondant à 298-300°C; α_{D} = +16,9°(c=1, DMF).

### Exemple 16: (-) Acide 4-[(1,2,3,4,4a,9b-Hexahydro-1,4a,9b-triméthyl 1,4-méthano dibenzofuranne -8-yl) carboxamido] benzoïque

La même synthèse que celle effectuée dans le cas de l'exemple **14** à partir de l'acide (+) [1,2,3,4,4a,9b-Hexahydro-1,4a,9b-triméthyl-1,4-méthano dibenzofuranne-8-yl] carboxylique, suivie d'une hydrogénation dans les mêmes conditions que pour l'exemple **15** conduit à l'énantiomère lévogyre **16** présentant les mêmes caractéristiques physico-chimiques que celles de l'énantiomère **15** dextrogyre; (α_{D} =-15,5°(c=1, CHCl3).

### Exemple 17: 4-[(1,2,3,4,4a,9b-Hexahydro-1,2,3,4-Tétrahydro-1,4a,9b-triméthyl - 1,4-méthano dibenzofuranne-8-yl) thiocarboxamido] benzoate de benzyle.

1,97g (4,1mmoles) de l'amide obtenue à l'exemple **14** dans 20ml de toluène sont traités par 0,83g (2mmoles) de réactif de Lawesson et sont chauffés à 110°C pendant 2heures30. Après évaporation à sec, on effectue une chromatographie sur silice (hexane/dichlorométhane,20:80) .On isole alors 1,94g (95%) du dérivé **17** sous la forme d'un solide jaune amorphe.

### Exemple 18: (+) Acide 4-[(1,2,3,4,4a,9b-Hexahydro-1,2,3,4-tétrahydro-1,4a,9b-triméthyl-1,4-méthano dibenzofuranne-8-yl) thiocarboxamido]benzoïque.

L'ester benzylique **17** (1,92g, 3,8mmoles) dans 50ml de méthanol est saponifié en présence de 2g de soude. On chauffe à reflux pendant 3 heures 30. Après le même traitement qu'à l'exemple **3**, suivi d'une recristallisation dans le mélange acétate d'éthyle-hexane, on isole 1,25g (79%) de dérivé **18** sous forme d'un solide jaune fondant à 234-236°C; α_{D} = +29°(c=1, CHCl3).

### Exemple 19 : (-) Acide 4-[(1,2,3,4,4a,9b-Hexahydro-1,2,3,4-Tétrahydro-1,4a,9b-triméthyl-1,4-méthano dibenzofuranne-8-yl) thiocarboxamido] benzoïque.

La même synthèse que celle effectuée dans le cas de l'exemple **17** à partir de l'acide (+) [1,2,3,4,4a,9b-Hexahydro-1,2,3,4-Tétrahydro-1,4a,9b-triméthyl-1,4-méthano dibenzofuranne-8-yl] carboxylique, suivie d'une saponification dans les mêmes conditions que pour l'exemple **18** conduit à l'énantiomère lévogyre **19** présentant les mêmes caractéristiques physico-chimiques que celles de l'énantiomère dextrogyre **(18)**; α_{D} = -25°(c=1, DMF).

### Exemple 20: 2-Hydroxy-4-[(1,2,3,4-tétrahydro-1,4a,9b-triméthyl-1,4-méthano dibenzofuranne-8-yl) carbonyloxy] benzoate de benzyle.

0,58mg (2 mmoles) de chlorure de l'acide (+)[1,2,3,4,4a,9b-Hexahydro-1,2,3,4-tétrahydro-1,4a,9b-triméthyl-1,4-méthanodibenzofuranne -8-yl] carboxylique en solution dans 10ml de THF sont condensés avec du de 2,4-dihydroxybenzoate de benzyle (0,48g, 2 mmoles) dans les conditions décrites à l'exemple **11**. On isole après le même traitement suivi d'une chromatographie (heptane/dichlorométhane, 20:80) 0,39g (40%) du dérivé **20** sous forme d'une huile jaune.

### Exemple 21: (+) Acide 2-hydroxy-4-[(1,2,3,4,4a,9b-Hexahydro-1,2,3,4-tétrahydro -1,4a,9b-triméthyl-1,4-méthano dibenzofuranne -8-yl) carbonyloxy] benzoïque.

L'ester 29 (0,38g, 0,76 mmoles) dans 50ml de dioxanne est hydrogéné en présence de 80mg de palladium sur charbon (10%) sous une préssion d'hydrogène de 5 bars pendant 2 heures 30 à température ambiante. Après le même traitement qu'à l'exemple **12** suivi d'une recristallisation dans un mélange acétate d'éthyle/heptane,on isole 0,18g (58%) du dérivé **21** sous forme de cristaux blancs, fondant à 208-210°C; α_{D} = +3,6° (c=1, DMF).

### Exemple 22: 2-Hydroxy -4-[(1,2,3,4-tétrahydro-1,4a,9b-triméthyl-1,4-méthano dibenzofuranne -8-yl) carboxamido] benzoate de benzyle.

1,32g (4,5 mmoles) du chlorure de l'acide (+) [1,2,3,4-tétrahydro-1,4a,9b-triméthyl-1,4-méthanodibenzofuranne -8-yl] carboxylique dans 20ml de THF sont traités par une solution contenant 0,76g (4,5 mmoles) de 2-hydroxy -4-aminobenzoate de méthyle et 0,64ml de triéthylamine dans 15ml de THF. Le milieu réactionnel est laissé sous agitation à température ambiante pendant 5 heures. On verse ensuite le milieu réactionnel dans l'eau et extrait à l'éther éthylique. Le traitement habituel suivi d'une chromatographie (dichlorométhane/heptane, 80:20) conduit à 1,16g (60%) du dérivé **22** sous forme de cristaux blancs, fondant à 231-233°C.

### Exemple 23: (-) Acide 2-hydroxy-4-[(1,2,3,4,4a,9b-Hexahydro-1,4a,9b-triméthyl - 1,4-méthano dibenzofuranne -8-yl) carboxamido] benzoïque.

Le dérivé **22** (0,67mg, 1 mmole) est saponifié par par 5ml d'une solution méthanolique de soude 2N à reflux pendant 3heures. Après le même traitement qu'à l'exemple **4**, suivi d'une chromatographie sur silice (dichlorométhane/THF, 95/5), on isole 0,2g (51%) du dérivé **23** sous forme d'un solide blanc cristallin, fondant à 235-236°C; α_{D} =-14,2°(c=1, DMF).

### Exemple 24: 2-Hydroxy-4-[(1,2,3,4,4a,9b-Hexahydro-1,4a,9b-triméthyl-1,4-méthano dibenzofuranne-8-yl) thiocarboxamido] benzoate de méthyle.

0,93g (2,2 mmoles) de l'amide **22** dans 20ml de toluène sont traités par 0,46g de réactif de Lawesson dans les conditions décrires à l'exemple **17** pour conduire après le même traitement, suivi par une chromatographie sur silice (dichlorométhane/heptane, 9/1) à 1,15g (97%) du dérivé **24** sous forme de solide orange amorphe.

### Exemple 25: (-) Acide 2-hydroxy-4-[(1,2,3,4,4a,9b-Hexahydro-1,4a,9b-triméthyl - 1,4-méthano dibenzofuranne -8-yl) thiocarboxamido] benzoïque.

L'ester méthylique **24** (1,13g, 2,6mmoles) est saponifié dans les conditions décrites à l'exemple **18**. On isole après le même traitement , suivi d'une chromatographie sur silice (dichlorométhane/acétate d'éthyle, 9:1) et d'une recristallisation dans l'heptane 0,58g (53%) du dérivé **25** sous forme de cristaux jaunes fondant à 213-214°C; α_{D} = -22,8°(c=1, DMF).

### B. EXEMPLES DE FORMULATION

### 1) VOIE ORALE

(a) On prépare la composition suivante sous la forme d'un comprimé de 0,8 g

| | |
|---|---|
| Composé de l'exemple 9 | 0,005 g |
| Amidon prégélatinisé | 0,265 g |
| Cellulose microcristalline | 0,300 g |
| Lactose | 0,200 g |
| Stéarate de magnésium | 0,030 g |

Pour le traitement de l'acné, on administre à un individu adulte 1 à 3 comprimés par jour pendant 3 à 6 mois selon la gravité du cas traité.
(b) On prépare une suspension buvable, destinée à être conditionnée en ampoules de 5 ml

| | |
|---|---|
| Composé de l'exemple 10 | 0,050 g |
| Glycérine | 0,500 g |
| Sorbitol à 70 % | 0,500 g |
| Saccharinate de sodium | 0,010 g |
| Parahydroxybenzoate de méthyle | 0,040 g |
| Arôme q.s. | |
| Eau purifiée q.s.p. | 5 ml |

Pour le traitement de l'acné, on administre à un individu adulte 1 ampoule par jour pendant 3 mois selon la gravité du cas traité.
(c) On prépare la formulation suivante destinée à être conditionnée en gélules :

| | |
|---|---|
| Composé de l'exemple 15 | 0,025 g |
| Amidon de maïs | 0,060 g |
| Lactose q.s.p | 0,300 g |

Les gélules utilisées sont constituées de gélatine, d'oxyde de titane et d'un conservateur.
Dans le traitement du psoriasis, on administre à un individu adulte, 1 gélule par jour pendant 30 jours.

### 2) VOIE TOPIQUE

(a) On prépare la crème Eau-dans l'Huile non ionique suivante :

| | |
|---|---|
| Composé de l'exemple 9 | 0,100 g |
| Mélange d'alcools de lanoline émulsifs, de cires et d'huiles raffinés, vendu par la Société BDF sous la dénomination "Eucérine anhydre" | 39,900 g |
| Parahydroxybenzoate de méthyle | 0,075 g |
| Parahydroxybenzoate de propyle | 0,075 g |
| Eau déminéralisée stérile q.s.p | 100,000 g |

Cette crème est appliquée sur une peau psoriatique 1 à 2 fois par jour pendant 30 jours.
(b) On prépare un gel en réalisant la formulation suivante :

| | |
|---|---|
| Composé de l'exemple 9 | 0,050 g |
| Erythromycine base | 4,000 g |
| Butylhydroxytoluène | 0,050 g |
| Hydroxypropylcellulose vendue par la société Hercules sous le nom de "KLUCEL HF" | 2,000 g |
| Ethanol (à 95°) q.s.p. | 100,000 g |

Ce gel est appliqué sur une peau atteinte de dermatose ou une peau acnéique 1 à 3 fois par jour pendant 6 à 12 semaines selon la gravité du cas traité.
(c) On prépare une lotion antiséborrhéique en procédant au mélange des ingrédients suivants :

| | |
|---|---|
| Composé de l'exemple 10 | 0,030 g |
| Propylène glycol | 5,000 g |
| Butylhydroxytoluène | 0,100 g |
| Ethanol (à 95°) q.s.p. | 100,000 g |

Cette lotion est appliquée deux fois par jour sur un cuir chevelu séborrhéique et on constate une amélioration significative dans un délai compris entre 2 et 6 semaines.
(d) On prépare une composition cosmétique contre les effets néfastes du soleil en procédant au mélange des ingrédients suivants :

| | |
|---|---|
| Composé de l'exemple 12 | 1,000 g |
| Benzylidène camphre | 4,000 g |
| Triglycérides d'acides gras | 31,000 g |
| Monostéarate de glycérol | 6,000 g |
| Acide stéarique | 2,000 g |
| Alcool cétylique | 1,200 g |
| Lanoline | 4,000 g |
| Conservateurs | 0,300 g |
| Propylène glycol | 2,000 g |
| Triéthanolamine | 0,500 g |
| Parfum | 0,400 g |
| Eau déminéralisée q.s.p. | 100,000 g |

Cette composition est appliquée quotidiennement, elle permet de lutter contre le vieillissement photoinduit.
(e) On prépare la crème Huile dans l'Eau non ionique suivante :

| | |
|---|---|
| Composé de l'exemple 15 | 0,500 g |
| Vitamine D3 | 0,020 g |
| Alcool cétylique | 4,000 g |
| Monostéarate de glycérol | 2,500 g |
| Stéarate de PEG 50 | 2,500 g |
| Beurre de Karité | 9,200 g |
| Propylène glycol | 2,000 g |
| Parahydroxybenzoate de méthyle | 0,075 g |
| Parahydroxybenzoate de propyle | 0,075 g |
| Eau déminéralisée stérile q.s.p | 100,000 g |

Cette crème est appliquée sur une peau psoriatique 1 à 2 fois par jour pendant 30 Jours.
(f) On prépare un gel topique en procédant au mélange des ingrédients suivants :

| | |
|---|---|
| Composé de l'exemple 18 | 0,050 g |
| Ethanol | 43,000 g |
| a -tocophérol | 0,050 g |
| Polymère carboxyvinylique vendu sous la dénomination "Carbopol 941" par la société "Goodrich | 0,500 g |
| Triéthanolamine en solution aqueuse à 20 % en poids | 3,800 g |
| Eau | 9,300 g |
| Propylène glycol qsp | 100,000 g |

Ce gel est appliqué dans le traitement de l'acné 1 à 3 fois par jour pendant 6 à 12 semaines selon la gravité du cas traité.
(g) On prépare une lotion capillaire anti-chute et pour la repousse des cheveux en procédant au mélange des ingrédients suivants :

| | |
|---|---|
| Composé de l'exemple 19 | 0,05 g |
| Composé vendu sous la dénomination "Minoxidil" | 1,00 g |
| Propylène glycol | 20,00g |
| Ethanol | 34,92 g |
| Polyéthylèneglycol (masse moléculaire = 400) | 40,00 g |
| Butylhydroxyanisole | 0,01 g |
| Butylhydroxytoluène | 0,02 g |
| Eau qsp | 100,00 g |

On applique cette lotion 2 fois par jour pendant 3 mois sur un cuir chevelu ayant subi une chute de cheveu importante.
(h) On prépare une crème anti-acnéique en procédant au mélange des ingrédients suivants :

| | |
|---|---|
| Composé de l'exemple 21 | 0,050 g |
| Acide rétinoïque | 0,010 g |
| Mélange de stéarates de glycérol et de polyéthylène glycol (75 moles) vendu sous le nom de "Gelot 64" . par la société "GATTEFOSSE" | 15,000 g |
| Huile de noyau polyoxyéthylénée à 6 moles d'oxyde d'éthylène vendue sous le nom de "Labrafil M2130 CS" par la société "GATTEFOSSE" | 8,000 g |
| Perhydrosqualène | 10,000 g |
| Conservateurs | qs |
| Polyéthylèneglycol (masse moléculaire = 400) | 8,000 g |
| Sel disodique de l'acide éthylène-diamine tétricétique | 0,050 g |
| Eau purifiée qsp | 100,000 g |

Cette crème est appliquée sur une peau atteinte de dermatose ou une peau acnéique 1 à 3 fois par jour pendant 6 à 12 semaines.
(i) On prépare une crème huile dans l'eau en réalisant la formulation suivante :

| | |
|---|---|
| Composé de l'exemple 21 | 0,020 g |
| 17-valérate de bétamethasone | 0,050 g |
| S-carboxyméthyl cystéine | 3,000 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de "Tween 20" par la société "ATLAS" | 1,800 g |
| Mélange de mono et distéarate de glycérol vendu sous la dénomination de "Géléol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812" par la société "DYNAMIT NOBEL" | 4,000 g |
| Triéthanolamine (99 % en poids) | 2,500 g |
| Eau q.s.p. | 100,000 g |

Cette crème est appliquée 2 fois par jour sur une peau atteinte de dermatose pendant 30 jours.
(j) On prépare la crème de type huile dans l'eau suivante :

| | |
|---|---|
| Acide lactique | 5,000 g |
| Composé de l'exemple 22 | 0,020 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de Tween 20" par la société "ATLAS" | 1,800 g |
| Mélange de mono et distéarate de glycérol vendu sous la dénomination de "Geleol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812" . par la société "DYNAMIT NOBEL" | 4,000 g |
| Eau q.s.p. | 100,000 g |

Cette crème est appliquée 1 fois par jour, elle aide à lutter contre le vieillissement qu'il soit photoinduit ou chronologique.

## Revendications

1. Composé de formule générale (I): 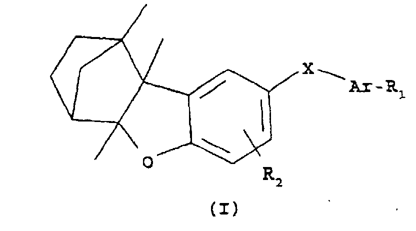 dans laquelle,
Ar représente l'un des radicaux suivants: 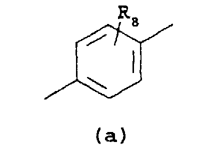 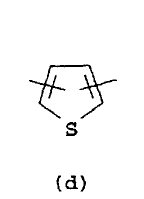 R₈ étant défini ci-dessous,
X représente les liaisons de formule (a)-(g) suivantes pouvant être lues dans les deux sens: 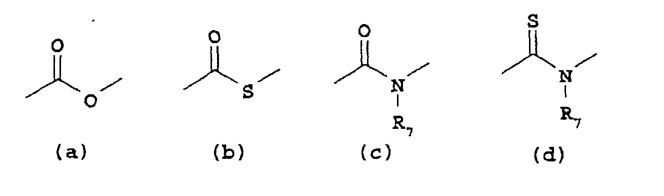 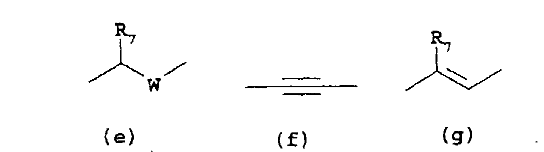 W et R₇ étant défini ci-dessous,
R₁ représente
(i) un atome d'hydrogène,
(ii) le radical -CH₃,
(iii) le radical -O-R₃
(iv) le radical -CH₂-O-R_{3,}
(v) le radical -O(CH₂)ₘ-(CO)ₙ-R₄,
(vi) le radical -CO-R₅
(vii) le radical -CO-O-R₆
R₂ représente un atome d'hydrogène ou le radical -(CH₂)ₙ-O-R₃,
m et n ainsi que R₃ à R₆ étant définis ci-après,
- W représente l'atome d'oxygène ou le radical N-R₇, R₇ étant défini ci-dessous,
- R₃ représente un atome d'hydrogène, un radical alkyle ayant de 1 à 12 atomes de carbone ou un radical
-CO-R₉, R₉ étant défini ci-dessous,
- R₄ représente un radical alkyle ayant de 1 à 12 atomes de carbone ou un hétérocycle,
- R₅ représente un atome d'hydrogène, un radical alkyle ayant de 1 à 12 atomes de carbone, ou un radical
-N (r',r''),
dans lequel, r' et r" identiques ou différents représentent un atome
d'hydrogène, un radical alkyle ayant de 1 à 12 atomes de carbone, un radical mono ou polyhydroxyalkyle, un radical phényle, éventuellement substitué par un ou plusieurs atomes d'halogène, une fonction hydroxyle ou une fonction nitro, ou un groupe méthoxy ou un reste d'amino acide, de peptide ou de sucre ou encore r' et r" pris ensemble forment un hétérocycle,
- R₆ représente un atome d'hydrogène, un radical alkyle linéaire ou ramifié ayant de 1 à 20 atomes de carbone, un radical alkényle, un radical mono ou polyhydroxyalkyle, un radical aryle ou aralkyle, un reste de sucre ou d'aminoacide ou de peptide,
- R₇ représente un atome d'hydrogène, un radical alkyle ayant de 1 à 12 atomes de carbone,
- R₈ représente le radical -(CH₂)ₙ-O-R₃, R₃ étant défini ci-dessus,
- R₉ représente un atome d'hydrogène, un radical alkyle ayant de 1 à 12 atomes de carbone, un radical aryle,
- m est un nombre entier égal à 1, 2 ou 3,
- n est un nombre entier égal à 0 ou 1,
et les isomères optiques et géométriques desdits composés purs, ou leurs mélanges en toutes proportions ainsi que leurs sels pharmaceutiquement acceptables.

2. Composé selon la revendication 1, caractérisé en ce qu'il répond à la formule générale (la): 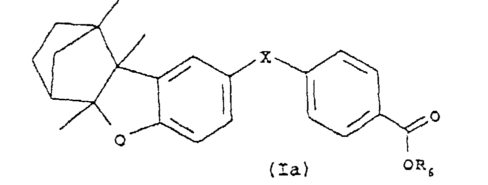 dans laquelle,
X a la signification (c), (d), (f) ou (g),
(c), (d), (f), (g) et R₆ étant définis dans la revendication 1.

3. Composé selon l'une des revendications 1 ou 2, caractérisé en ce qu'il est choisi parmi les composés suivants:
2-[(E)-2-(1,2,3,4,4a,9b-Hexahydro-1,4a,9b-triméthyl-1,4-méthanodibenzofuranne-8-yl)-1-propenyl] 4-thiophène carboxylate d'éthyle
Acide 2-[(E)2-(1,2,3,4,4a,9b-Hexahydro-1,4a,9b-triméthyl-1,4-méthanodibenzofuranne -8-yl)-1-propenyl] -4-thiophène carboxylique
Acide 2-[(E)2-(1,2,3,4,4a,9b-Hexahydro-1,4a,9b-triméthyl-1,4-méthanodibenzofuranne -8-yl)-1-propenyl] -5-thiophène carboxylique
4-[3-(1,2,3,4,4a,9b-Hexahydro-1,4a,9b-triméthyl-1,4-méthanodibenzofuranne -8-yl) éthynyl] benzoate de méthyle
Acide 4-[3-(1,2,3,4,4a,9b-Hexahydro-1,4a,9b-triméthyl-1,4-méthanodibenzofuranne -8-yl) éthynyl] benzoïque
Acide 4-[-2(1,2,3,4,4a,9b-Hexahydro-1,4a,9b-triméthyl-1,4-méthanodibenzofuranne -8-yl) éthynyl] -2-thiophène carboxylique.
4-[3-(1,2,3,4,4a,9b-Hexahydro-1,4a,9b-triméthyl-1,4-méthanodibenzofuranne -8-yl) carbonyloxy] benzoate de méthyle
Acide 4-[3-(1,2,3,4,4a,9b-Hexahydro-1,4a,9b-triméthyl-1,4-méthanodibenzofuranne -8-yl) carbonyloxy] benzoïque
4-[(1,2,3,4,4a,9b-Hexahydro-1,4a,9b-triméthyl-1,4-méthanodibenzofuranne -8-yl) carboxamido] benzoate de benzyle
Acide 4-[(1,2,3,4,4a,9b-Hexahydro-1,4a,9b-triméthyl-1,4-méthanodibenzofuranne-8-yl) carboxamido] benzoïque
4-[(1,2,3,4,4a,9b-Hexahydro-1,4a,9b-triméthyl-1,4-méthanodibenzofuranne -8-yl) carboxamido] -2-hydroxybenzoate de méthyle
Acide 4-[(1,2,3,4,4a,9b-Hexahydro-1,4a,9b-triméthyl-1,4-méthanodibenzofuranne-8-yl) carboxamido] -2-hydroxy benzoïque
Acide 4-[(1,2,3,4,4a,9b-Hexahydro-1,4a,9b-triméthyl-1,4-méthanodibenzofuranne-8-yl) thiocarboxamido] benzoate de méthyle
Acide 4-[(1,2,3,4,4a,9b-Hexahydro-1,4a,9b-triméthyl-1,4-méthanodibenzofuranne-8-yl) thiocarboxamido] benzoïque

4. A titre de médicament les composés de formule générale (I) selon l'une des revendications 1 à 3.

5. Utilisation d'un composé selon l'une des revendications 1 à 3 dans la fabrication d'un médicament destiné au traitement des affections dermatologiques liées à un désordre de la kératinisation portant sur la différenciation et sur la prolifération notamment les acnés vulgaires, comédoniennes, polymorphes, rosacées, les acnés nodulokystiques, conglobata, les acnés séniles, les acnés secondaires telles que l'acné solaire, médicamenteuse ou professionnelle; d'autres types de troubles de la kératinisation, notamment les ichtyoses, les états ichtyosiformes, la maladie de Darier, les kératodermies palmoplantaires, les leucoplasies et les états leucoplasiformes, le lichen cutané ou muqueux (buccal); d'autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale; de certaines affections inflammatoires ne présentant pas de trouble de la kératinisation; des proliférations dermiques ou épidermiques qu'elles soient bénignes ou malignes, qu'elles soient ou non d'origine virale telles que les verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires; d'autres désordres dermatologiques tels que les dermatoses bulleuses et les maladies du collagène; certains troubles ophtalmologiques, notamment les ,cornéopathies; du vieillissement de la peau, qu'il soit photoinduit ou chronologique, ou des pigmentations et des kératoses actiniques, ou toutes pathologies associées au vieillissement chronologique ou actinique; des stigmates de l'atrophie épidermique et/ou dermique induite par les corticostéroïdes locaux ou systémiques, ou tout autre forme d'atrophie cutanée, des troubles de la cicatrisation ou des vergetures; des troubles de la fonction sébacée tels que l'hyperséborrhée de l'acné ou la séborrhée simple; des états cancéreux ou précancéreux, plus particulièrement les leucémies promyélocytaires; d d'affections inflammatoires telles que l'arthrite, de toute affection d'origine virale au niveau cutané ou général; de l'alopécie; d'affections dermatologiques à composante immunitaire; d'affections du système cardiovasculaire telles que l'artériosclérose ou l'hypertension ainsi que le diabète non-insulino dépendant.

6. Composition pharmaceutique caractérisée en ce qu'elle comprend au moins un composé de formule générale (I) selon l'une des revendications 1 à 3 et un véhicule pharmaceutiquement acceptable.

7. Composition selon la revendication 6, caractérisée en ce que la concentration en composé(s) de formule générale (I) est comprise entre 0,001% et 5 % en poids par rapport au poids total de la composition.

8. Composition cosmétique caractérisée en ce qu'elle comprend un composé de formule générale (I) selon l'une des revendications 1 à 3 et un véhicule cosmétiquement acceptable.

9. Composition selon la revendication 8, caractérisée en ce que la concentration en composé(s) de formule générale (I) est comprise entre 0,001% et 3 % en poids par rapport au poids total de la composition.

## Patentansprüche

1. Verbindungen der allgemeinen Formel (I): 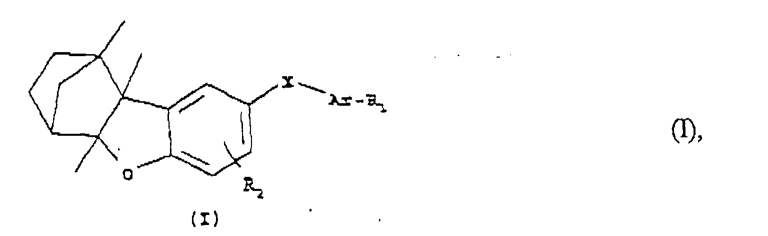 worin bedeuten:
Ar eine der folgenden Gruppen: 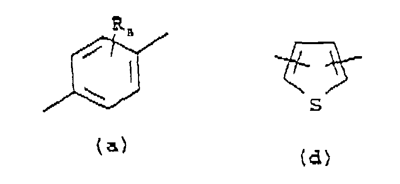 wobei R₈ die nachstehend angegebene Bedeutung aufweist;
X die folgenden Gruppen (a) - (g), wobei die angegebenen Formeln von links nach rechts und umgekehrt gelesen werden können: 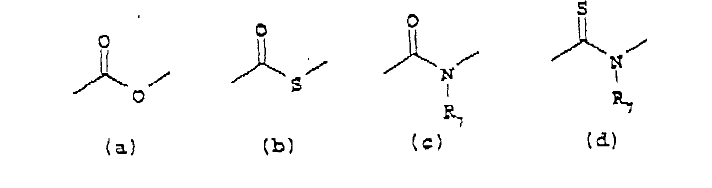 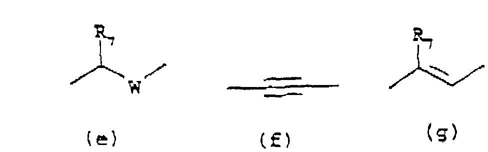 wobei W und R₇ die nachfolgend angegebenen Bedeutungen aufweisen;
R₁ :
(i) Wasserstoff,
(ii) die Gruppe -CH₃,
(iii) eine Gruppe -O-R₃,
(iv) eine Gruppe -CH₂-O-R₃,
(v) eine Gruppe -O(CH₂)ₘ-(CO)ₙ-R₄,
(vi) eine Gruppe -CO-R₅
(vii) eine Gruppe -CO-O-R₆;
R₂ ein Wasserstoffatom oder eine Gruppe (CH₂)ₙ-O-R₃, wobei m, n, R₃ bis R₆ nachstehend definiert sind; und
- W Sauerstoff oder eine Gruppe N-R₇, wobei R₇ nachstehend definiert ist;
- R₃ ein Wasserstoffatom, eine Alkylgruppe mit 1 bis 12 Kohlenstoffatomen oder eine Gruppe -CO-R₉, wobei R₉ nachstehend definiert ist;
- R₄ eine Alkylgruppe mit 1 bis 12 Kohlenstoffatomen oder einen Heterocyclus;
- R₅ ein Wasserstoffatom, eine Alkylgruppe mit 1 bis 12 Kohlenstoffatomen oder eine Gruppe -N(r',r"), worin r' und r", die identisch oder voneinander verschieden sind, Wasserstoff, eine Alkylgruppe mit 1 bis 12 Kohlenstoffatomen-, eine Mono-oder Polyhydroxyalkylgruppe, eine Phenylgruppe, die gegebenenfalls mit einem oder mehreren Halogenatomen, Hydroxy, Nitro oder Methoxy substituiert ist, oder einen Aminosäure-, Peptid- oder Zuckerrest; oder r' und r" bilden gemeinsam einen Heterocyclus;
- R₆ Wasserstoff, eine geradkettige oder verzweigte Alkylgruppe mit 1 bis 20 Kohlenstoffatomen, Alkenyl, Mono- oder Polyhydroxyalkyl, Aryl oder Aralkyl, einen Zucker-, Aminosäure- oder Peptidrest;
- R₇ ein Wasserstoffatom oder eine Alkylgruppe mit 1 bis 12 Kohlenstoffatomen;
- R₈ die Gruppe (CH₂)ₙ-O-R₃, wobei R₃ oben definiert wurde;
- R₉ ein Wasserstoffatom, eine Alkylgruppe mit 1 bis 12 Kohlenstoffatomen oder Aryl;
- m 1, 2 oder 3;
- n 0 oder 1;
sowie die optischen oder geometrischen Isomeren der reinen Verbindungen oder deren Gemische in beliebigen Anteilen sowie ihre pharmazeutisch akzeptablen Salze.

2. Verbindungen nach Anspruch 1, dadurch gekennzeichnet, daß sie der allgemeinen Formel (Ia) entsprechen: 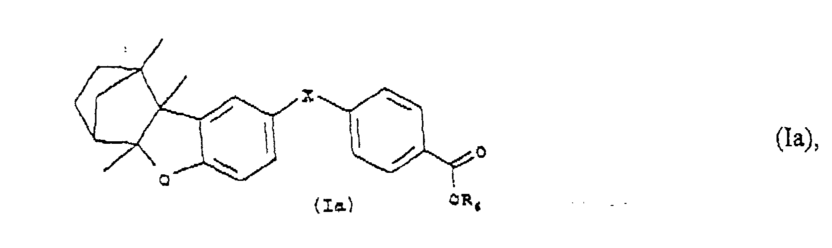 worin X (c), (d), (f) oder (g) bedeutet, wobei (c), (d), (f), (g) und R₆ die in Anspruch 1 angegebenen Bedeutungen aufweisen.

3. Verbindungen nach einem der Ansprüche 1 oder 2, dadurch gekennzeichnet, daß sie unter den folgenden Verbindungen ausgewählt sind;
- Ethyl-2-[(E)-2-(1,2,3,4,4a, 9b-hexahydro-1,4a,9b-trimethyl-1,4-methanodibenzofuran-8-yl)-1-propenyl]-4-thiophen-carboxylat;
- 2-[(E)-2-(1,2,3,4,4a,9b-Hexahydro-1,4a,9b-trimethyl-1,4-methanodibenzofuran-8-yl)-1-propenyl]-4-thiophen-carbonsäure;
- 2-[(E)-2-(1,2,3,4,4a,9b-Hexahydro-1,4a,9b-trimethyl-1,4-methanodibenzofuran-8-yl)-1-propenyl]-5-thiophen-carbonsäure;
- Methyl-4-[3-(1,2,3,4,4a,9b-hexahydro-1,4a,9b-trimethyl-1,4-methanodibenzofuran-8-yl)-ethinyl]-benzoat;
- 4-[3-(1,2,3,4,4a,9b-Hexahydro-1,4a,9b-trimethyl-1,4-methanodibenzofuran-8-yl)-ethinyl]-benzoesäüre;
- 4-[2-(1,2,3,4,4a,9b-Hexahydro-1,4a,9b-trimethyl-1,4-methanodibenzofuran-8-yl)-ethinyl]-2-thiophen-carbonsäure;
- Methyl-4-[3-(1,2,3,4,4a,9b-hexahydro-1,4a,9b-trimethyl-1,4-methanodibenzofuran-8-yl)-carbonyloxy]-benzoat;
- 4-[3-(1,2,3,4,4a,9b-Hexahydro-1,4a,9b-trimethyl-1,4-methanodibenzofuran-8-yl)-carbonyloxy]-benzoesäure;
- Benzyl-4-[(1,2,3,4,4a,9b-hexahydro-1,4a,9b-trimethyl-1,4-methano-dibenzofuran-8-yl)-carboxamido]-benzoat;
- 4-[(1,2,3,4,4a,9b-Hexahydro-1,4a,9b-trimethyl-1,4-methanodibenzofuran-8-yl)-carboxamido]-benzoesäure;
- Methyl 4-[(1,2,3,4,4a,9b-hexahydro-1,4a,9b-trimethyl-1,4-methanodibenzofuran-8-yl)-carboxamido]-2-hydroxy-benzoat;
- 4-[(1,2,3,4,4a,9b-Hexahydro-1,4a,9b-trimethyl-1,4-methanodibenzofuran-8-yl)-carboxamido]-2-hydroxy-benzoesäure;
- Methyl-4-[(1,2,3,4,4a,9b-hexahydro-1,4a,9b-trimethyl-1,4-methano-dibenzofuran-8-yl)-thiocarboxamido]-benzoat; und
- 4-[(1,2,3,4,4a,9b-Hexahydro-1,4a,9b-trimethyl-1,4-methanodibenzofuran-8-yl)-thiocarboxamido]-benzoesäure;

4. Verbindungen der allgemeinen Formel (I) nach einem der Ansprüche 1 bis 3 als Arzneimittel.

5. Verwendung einer Verbindung nach einem der Ansprüche 1 bis 3 zur Herstellung eines Arzneimittel, das zur Behandlung von dermatologischen Erkrankungen, die mit einer Verhornungsstörung verbunden sind, welche auf der Differenzierung und Proliferation beruht, insbesondere zur Behandlung von Acne vulgaris, Acne comedonica, polymorpher Acne, Acne rosacea, nodulocystischer Acne, Acne conglobata, Acne senilis und sekundären Acneformen, wie Acne solaris, Acne medikamentosa oder Acne professionalis; weiterer Störungen der Keratinisierung, insbesondere zur Behandlung von Ichtyosis, ichtyosisartigen Zuständen, der Darier Kankheit, Palmoplantarkeratosen, Leukoplakien und leucoplakieformen Zuständen und Lichen der Haut oder der Schleimhäute (buccalis); weiterer dermatologischer Erkrankungen, die mit einer Störung der Keratinisierung mit einer entzündlichen und/oder immunoallergischen Komponente verbunden sind, und insbesondere beliebiger Formen der Psoriasis der Haut, der Schleimhäute oder der Nägel, Psoriasis arthropathica oder auch der Atopie der Haut wie Ekzemen oder Atopie der Atemwege oder auch Hypertrophie des Zahnfleisches; Proliferationen der Dermis oder Epidermis, die gutartig oder bösartig und die gegebenenfalls viralen Ursprungs sein können, wie Verrucae vulgares, Verrucae planae und Epidermodysplasia verruciformis, Papillomatosis oralis oder florida, und Proliferationen, die durch UV-Strahlung hervorgerufen werden können, insbesondere im Falle von Epithelioma basocellulare und spinocellulare; weiterer dermatologischer Störungen, wie bullösen Dermatosen und Erkrankungen des Kollagens; verschiedener ophthalmologischer Störungen, insbesondere von Corneopathien; zur Behebung oder zur Bekämpfung der Hautalterung, die lichtinduziert oder altersbedingt sein kann, oder zur Verminderung der Pigmentierung und der aktinischen Keratosen oder beliebiger Erkrankungen, die mit der altersbedingten oder aktinischen Hautalterung verbunden sind; Wundmalen der epidermalen und/oder dermalen Atrophie, die durch lokal oder systemisch verabreichte Corticosteroide hervorgerufen werden, oder beliebiger weiterer Formen der Atrophie der Haut; Störungen der Wundheilung oder Streifen; Funktionsstörungen der Talgdrüsen, wie Hyperseborrhoe bei Acne oder Seborrhoe simplex; von krebsartigen oder präcancerösen Zuständen, insbesondere promyelocytärer Leukämie; entzündlichen Erkrankungen, wie Arthritis; beliebiger Hautkrankheiten viralen Ursprungs oder allgemeinen Erkrankungen viralen Ursprungs; dermatologischen Erkrankungen mit immunologischer Komponente; und Erkrankungen der cardiovaskulären Systems, wie Arteriosklerose oder Bluthochdruck sowie nicht insulinpfichtiger Diabetis, vorgesehen ist.

6. Pharmazeutische Zusammensetzung, dadurch gekennzeichnet, daß sie mindestens eine Verbindung der allgemeinen Formel (I) nach einem der Ansprüche 1 bis 3 und einen pharmazeutisch akzeptablen Träger enthält.

7. Zusammensetzung nach Anspruch 6, dadurch gekennzeichnet, daß die Konzentration der Verbindung(en) der allgemeinen Formel (I) im Bereich von 0,001 bis 5 Gew.-%, bezogen auf das Gesamtgewicht der Zusammensetzung, liegt.

8. Kosmetische Zusammensetzung, dadurch gekennzeichnet, daß sie mindestens eine Verbindung der allgemeinen Formel (I) nach einem der Ansprüche 1 bis 3 und einen kosmetisch akzeptablen Träger enthält.

9. Zusammensetzung nach Anspruch 8, dadurch gekennzeichnet, daß die Konzentration der Verbindung(en) der allgemeinen Formel (I) im Bereich von 0,001 bis 3 Gew.-%, bezogen auf das Gesamtgewicht der Zusammensetzung, liegt.

## Claims

1. Compound of general formula (I): 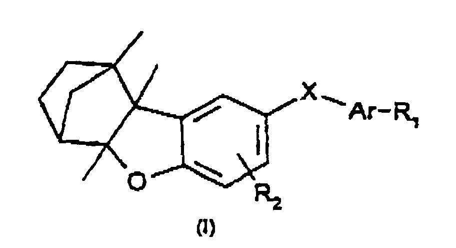 in which
Ar represents one of the following radicals: 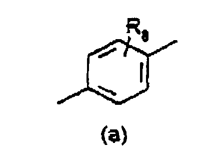 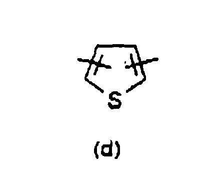 R₈ being defined hereinbelow,
X represents the following bonds of formula (a)-(g), which can be read in both directions: 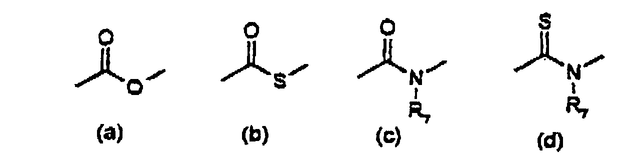 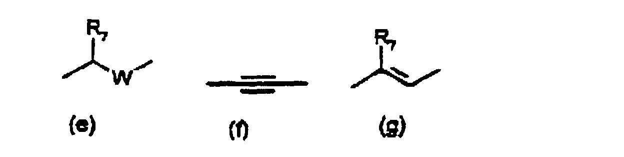 W and R₇ being defined hereinbelow,
R₁ represents
(i) a hydrogen atom,
(ii) the -CH₃ radical,
(iii) the -O-R₃ radical,
(iv) the -CH₂-O-R₃ radical,
(v) the -O(CH₂)ₘ-(CO)ₙ-R₄ radical,
(vi) the -CO-R₅ radical,
(vii) the -CO-O-R₆ radical,
R₂ represents a hydrogen atom or the -(CH₂)ₙ-O-R₃ radical,
m and n as well as R₃ to R₆ being defined below,
- W represents the oxygen atom or the N-R₇ radical, R₇ being defined hereinbelow,
- R₃ represents a hydrogen atom, an alkyl radical having from 1 to 12 carbon atoms or a -CO-R₉ radical, R₉ being defined hereinbelow,
- R₄ represents an alkyl radical having from 1 to 12 carbon atoms or a heterocycle,
- R₅ represents a hydrogen atom, an alkyl radical having from 1 to 12 carbon atoms or an -N(r',r'') radical,
in which r' and r", which are identical or different, represent a hydrogen atom, an alkyl radical having from 1 to 12 carbon atoms, a mono- or polyhydroxyalkyl radical, a phenyl radical, optionally substituted by one or more halogen atoms, a hydroxyl functional group or a nitro functional group, or a methoxy group, or an amino acid, peptide or sugar residue or alternatively r' and r'', taken together, form a heterocycle,
- R₆ represents a hydrogen atom, a linear or branched alkyl radical having from 1 to 20 carbon atoms, an alkenyl radical, a mono- or polyhydroxyalkyl radical, an aryl or aralkyl radical, or a sugar or amino acid or peptide residue,
- R₇ represents a hydrogen atom or an alkyl radical having from 1 to 12 carbon atoms,
- R₈ represents the -(CH₂)ₙ-O-R₃ radical, R₃ being defined hereinabove,
- R₉ represents a hydrogen atom, an alkyl radical having from 1 to 12 carbon atoms or an aryl radical,
- m is an integer equal to 1, 2 or 3,
- n is an integer equal to 0 or 1,
and the optical and geometrical isomers of the said pure compounds or their mixtures in any proportion and their pharmaceutically acceptable salts.

2. Compound according to Claim 1, characterized in that it corresponds to the general formula (Ia): 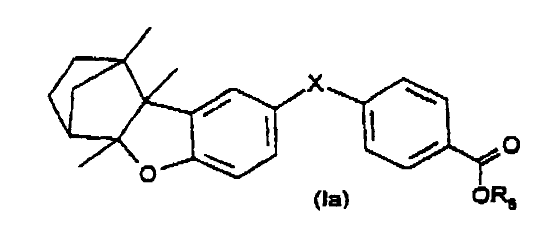 in which
X has the meaning (c), (d), (f) or (g),
(c), (d), (f), (g) and R₆ being defined in Claim 1.

3. Compound according to either of Claims 1 and 2, characterized in that it is chosen from the following compounds:
Ethyl 2-[(E)-2-(1,2,3,4,4a,9b-hexahydro-1,4a,9b-trimethyl-1,4-methanodibenzofuran-8-yl)-1-propenyl]-4-thiophenecarboxylate
2-[(E)-2-(1,2,3,4,4a,9b-Hexahydro-1,4a,9b-trimethyl-1,4-methanodibenzofuran-8-yl)-1-propenyl]-4-thiophene-carboxylic acid
2-[(E)-2-(1,2,3,4,4a,9b-Hexahydra-1,4a,9b-trimethyl-1,4-methanodibenzofuran-8-yl)-1-propenyl]-5-thiophene-carboxylic acid
Methyl 4-[3-(1,2,3,4,4a,9b-hexahydro-1,4a,9b-trimethyl-1,4-methanodibenzofuran-8-yl)ethynyl]benzoate
4-[3-(1,2,3,4,4a,9b-Hexahydro-1,4a,9b-trimethyl-1,4-methanodibenzofuran-8-yl)ethynyl]benzoic acid
4-[2-(1,2,3,4,4a,9b-Hexahydro-1,4a,9b-trimethyl-1,4-methanodibenzofuran-8-yl)ethynyl]-2-thiophenecarboxylic acid
Methyl 4-[3-(1,2,3, 4,4a,9b-hexahydro-1,4a,9b-trimethyl-1,4-methanodibenzofuran-8-yl)carbonyloxylbenzoate
4-[3-(1,2,3,4,4a,9b-Hexahydro-1,4a,9b-trimethyl-1,4-methanodibenzofuran-8-yl)carbonyloxy]benzoic acid
Benzyl 4-[(1,2,3,4,4a,9b-hexahydro-1,4a,9b-trimethyl-1,4-methanodibenzofuran-8-yl) carboxamido]benzoate
4-[(1,2,3,4,4a,9b-Hexahydro-1,4a,9b-trimethyl-1,4-methanodibenzofuran-8-yl)carboxamido]benzoic acid
Methyl 4-[(1,2,3,4,4a,9b-hexahydro-1,4a,9b-trimethyl-1,4-methanodibenzofuran-8-yl)carboxamido]-2-hydroxy-benzoate
4-[(1,2,3,4,4a,9b-Hexahydro-1,4a,9b-trimethyl-1,4-methanodibenzofuran-8-yl)carboxamido]-2-hydroxybenzoic acid
Methyl 4-[(1,2,3,4,4a,9b-hexahydro-1,4a,9b-trimethyl-1,4-methanodibenzofuran-8-yl)thiocarboxamido]benzoate
4-[(1,2,3,4,4a,9b-Hexahydro-1,4a,9b-trimethyl-1,4-methanodibenzofuran-8-yl)thiocarboxamido]benzoic acid.

4. As medicament, the compounds of general formula (I) according to one of Claims 1 to 3.

5. Use of a compound according to one of Claims 1 to 3 in the manufacture of a medicament intended for the treatment of dermatological conditions linked to a disorder of keratinization involving differentiation and proliferation, in particular acne vulgaris, comedonic or polymorphic acne, acne rosacea, nodulocystic acne, acne conglobata, senile acne and secondary acnes such as solar, drug or occupational acne; other types of disorders of keratinization, in particular ichthyoses, ichthyosiform conditions, Darier's disease, palmoplantar keratoderma, leucoplakia and leucoplakiform conditions or cutaneous or mucosal (oral) lichen; other dermatological conditions linked to a disorder of keratinization with an inflammatory and/or immunoallergic component and, in particular, all forms of psoriasis, either cutaneous, mucosal or ungual, and even psoriatic rheumatism, or alternatively cutaneous atopy, such as eczema, or respiratory atopy or alternatively gingival hypertrophy; certain inflammatory conditions which do not show disorder of keratinization; dermal or epidermal proliferations, whether they are benign or malignant and whether they are or are not of viral origin, such as common warts, flat warts and epidermodysplasia verruciformis, florid or oral papillomatoses and the proliferations which can be induced by ultraviolet radiation, in particular in the case of basal cell and prickle cell epithelioma; other dermatological disorders, such as bullous dermatoses and collagen diseases; certain ophthalmological disorders, in particular corneopathies; ageing of the skin, whether photoinduced or chronologic, or actinic keratoses and pigmentations or any pathology associated with chronologic or actinic ageing; stigmata of epidermal and/or dermal atrophy induced by local or systemic corticosteroids, or any other form of cutaneous atrophy, disorders of cicatrization or stretch marks; disorders of the sebaceous function such as hyperseborrhoea of acne or simple seborrhoea; cancerous or precancerous conditions, more particularly promyelocytic leukaemias; inflammatory conditions such as arthritis, any condition of viral origin at the cutaneous or general level; alopecia; dermatological conditions with an immune component; ailments of the cardiovascular system such as arteriosclerosis or hypertension, as well as non-insulin-dependent diabetes.

6. Pharmaceutical composition, characterized in that it comprises at least one compound of general formula (I) according to one of Claims 1 to 3 and a pharmaceutically acceptable vehicle.

7. Composition according to Claim 6, characterized in that the concentration of compound(s) of general formula (I) is between 0.001% and 5% by weight with respect to the total weight of the composition.

8. Cosmetic composition, characterized in that it comprises a compound of general formula (I) according to one of Claims 1 to 3 and a cosmetically acceptable vehicle.

9. Composition according to Claim 8, characterized in that the concentration of compound(s) of general formula (I) is between 0.001% and 3% by weight with respect to the total weight of the composition.
